# EUROPEAN PATENT APPLICATION

(11) **EP 0 945 492 A1**
(43) Date of publication of application: **29.09.1999**
(21) Application number: 97946127.4
(22) Date of filing: 05.12.1997
(51) Int. Cl.: C08L 101/06, C08G 75/14, C08L 33/14, C09D 201/06, C09D 133/14, C09J 201/06, C09J 133/14, C07D 327/04

(54) **AQUEOUS DISPERSION COMPOSITION**

(30) Priority: 11.12.1996 JP 33054096
(71) Applicant: Kyowa Yuka Co., Ltd., Tokyo 100-8185 (JP)
(72) Inventor: JIMBO, Shinichiro, Tokyo 153 (JP); MATSUSHITA, Shoshiro, Mie 512 (JP); SHIMIZU, Ikuo, Mie 512 (JP); HOTTA, Iwao, Mie 510 (JP); IKUTA, Masanori, Mie 510 (JP); ITANI, Izumi, Mie 510 (JP)
(74) Representative: VOSSIUS & PARTNER
(86) International application number: JP9704471
(87) International publication number: WO9826005

(57) **Abstract**

The present invention provides a composition which is effective for use in paints, adhesives, inks, and the like, comprising an aqueous dispersion of a compound containing at least one 5-membered ring dithiocarbonate group, expressed by the following general formula (I): 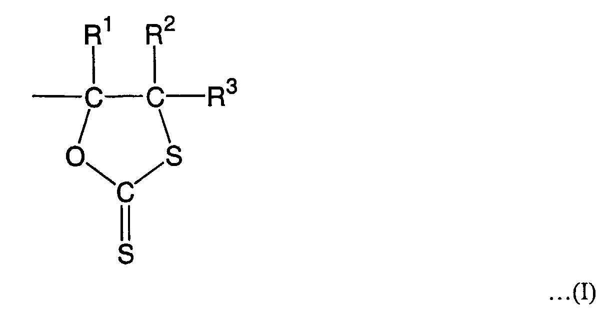 (wherein R¹, R² and R³ are the same or different, and represent a hydrogen atom or a lower alkyl group) and an ether bond; and an amino compound.

## Description

### Technical Field

The present invention relates to an aqueous dispersion composition comprising a compound containing a dithiocarbonate group, and an amino compound, which is effective for use in paints, adhesives, inks, and the like.

### Background Art

An aqueous dispersion that contains a reactive, functional group in its molecule is effective for various uses. For example, such an aqueous dispersion can provide paints, adhesives, inks, and the like, possessing superior hardness, strength, water resistance, chemical resistance, stamp resistance, blocking resistance, and the like.

Various compositions are known as a reactive, aqueous dispersion. Examples include vinyl and acrylic aqueous polymer containing a 1,3-dioxolan-2-on-4-yl group (disclosed in Japanese Published Unexamined Patent Application No. 325539/92); and an aqueous dispersion composition, comprising an epoxy resin and a compound containing an active hydrogen (disclosed in Japanese Published Unexamined Patent Application No. 293953/92). However, the aforementioned reactive, aqueous dispersions are not necessarily adequate for practical use, from the standpoint of their properties such as water resistance, chemical resistance, and blocking resistance, under the drying conditions at 60°C or below.

In addition, as a polymer containing 5-membered ring dithiocarbonate (1,3-oxathiolane-2-thione) groups, a homopolymer of 5-(methacryloyl) methyl-1,3-oxathiolane-2-thione is disclosed in Japanese Published Unexamined Patent Application No. 247027/93, and a copolymer of 5-(methacryloyl) methyl-1,3-oxathiolane-2-thione and methyl methacrylate is disclosed in the Journal of Polymer Science: Part A; Polymer Chemistry, 33, 1005(1995); however, there is no disclosure with regard to an aqueous dispersion thereof. Additionally, Macromolecules, 28, 5386 (1995) discloses a bisphenol A-type compound containing two 1,3-oxathiolane-2-thione groups, and the like; however, there exists no disclosure with regard to an aqueous dispersion thereof.

Furthermore, an aqueous emulsion comprising a copolymer of a vinyl monomer containing a 5-(methacryloyl)methyl-1,3-oxathiolane-2-thione group, and a (meth)acrylic acid ester, is disclosed in Japanese Published Unexamined Patent Application No. 62190/95.

### Disclosure of the Invention

It is an object of the present invention to provide a composition comprising an aqueous dispersion of a compound containing at least one 5-membered ring dithiocarbonate group, expressed by the following general formula (I): 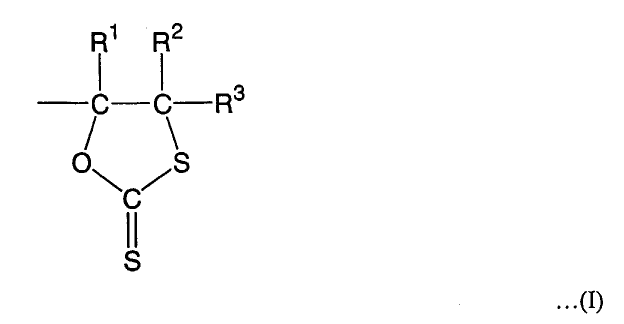 (wherein R¹, R² and R³ are the same or different, and represent a hydrogen atom or a lower alkyl group) and an ether bond; and an amino compound.

In the definition of the general formula (I), the lower alkyl group represents a straight or branched chain alkyl group with 1 to 4 carbon atoms, such as methyl, ethyl, propyl, isopropyl, butyl, isobutyl, tert-butyl or the like.

The dithiocarbonate compound, a component of the composition according to the present invention, contains an ether bond within its molecule, such that the resin, obtained after the composition cures, exhibits a superior flexibility and chemical resistance.

Examples of the compound containing at least one 5-membered ring dithiocarbonate group and an ether bond, in the present invention, include a compound, obtained by means of reacting an oxyrane compound such as a diphenylepoxy resin, ethylene oxide, propylene oxide, butylene oxide, triglycidyl isocyanurate, epoxidated soybean oil, epoxidated soybean oil fatty acid, or the like, with carbon disulfide; and a compound, obtained by means of reacting a glycidyl ether with carbon disulfide, the glycidyl ether having been obtained by means of reacting a polyhydric alcohol such as ethylene glycol, diethylene glycol, triethylene glycol, polyethylene glycol, 1,2-propylene glycol, 1,3-propylene glycol, polypropylene glycol, 1,3-butanediol, 1,4-butanediol, 1,2-butanediol, 1,5-pentanediol, 1,6-hexanediol, neopentyl glycol, cyclohexane dimethanol, 3-methy- 1,5-pentanediol, 2,4-diethyl-1,5-pentanediol, 1,9-nonanediol, 1,3-octanediol, trimethylolpropane, trimethylolethane, glycerin, or the like, with epichlorohydrin.

Additionally, with regard to the dithiocarbonate compound, which is a component of the composition according to the present invention, a compound further containing phenylene or cyclohexylene within its molecule is preferably used, due to its superior water resistance, chemical resistance, and properties at a high temperature.

Furthermore, examples of the compound containing phenylene or cyclohexylene within the molecule include compounds, obtained by means of reacting a glycidyl ether with carbon disulfide, and the like. In this case, the glycidyl ether can be obtained by means of reacting a phenol compound, such as resorcinol, hydroquinone, pyrocatechol, bisphenol A, dihydroxydiphenylmethane (bisphenol F), bisphenol S, tetrabromobisphenol A, 4,4-dihydroxydiphenylcyclohexane, 4,4-dihydroxy-3,3-dimethyldiphenylmethane, 4,4-dihydroxybenzophenone, tris(4-hydroxyphenyl)methane, bis(4-hydroxyphenyl)ether, novolakphenol, novolakcresol, bis (4-hydroxyphenyl)sulfone, bis (3,5-dimethyl-4-hydroxyphenyl)sulfone, or the like, a hydrogenated compound thereof, or a halide thereof, with epichlorohydrin. Among the aforementioned, preferred compounds are those expressed by the following general formula (II): 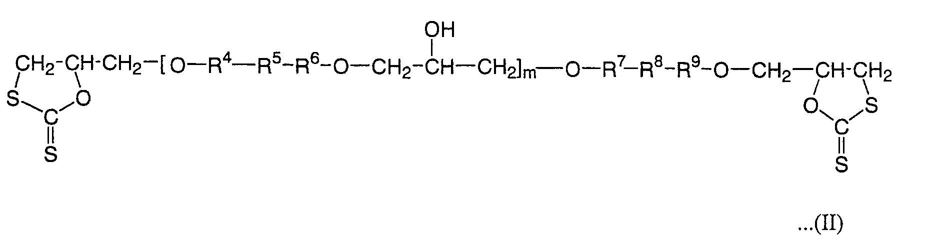 (wherein R⁴, R⁶, R⁷ and R⁹ are the same or different, and represent phenylene or cyclohexylene, in which 1 to 4 hydrogen atoms may be substituted with Br; R⁵ and R⁸ are the same or different, and represent methylene, C(CH₃)₂, or S; and m represents an integer between 1 and 40). Furthermore, more preferably used is a compound expressed by the general formula (II), wherein R⁴, R⁶, R⁷ and R⁹ represent the same group, and R⁵ and R⁸ represent the same group. Furthermore, even more preferably used are the following compounds: a compound expressed by the general formula (II), wherein R⁴, R⁶, R⁷ and R⁹ each represent phenylene, and R⁵ and R⁸ each represent methylene; a compound expressed by the general formula (II), wherein R⁴, R⁶, R⁷ and R⁹ each represent cyclohexylene, and R⁵ and R⁸ each represent C(CH₃)₂; or a compound expressed by the general formula (II), wherein R⁴, R⁶, R⁷ and R⁹ each represent cyclohexylene, and R⁵ and R⁸ each represent methylene. The compound expressed by the general formula (II) is superior in adhesiveness, since hydrophilic hydroxyl groups and hydrophobic hydrocarbon groups are systematically distributed throughout each molecule.

With regard to the amino compound, which is a component of the composition according to the present invention, a primary or secondary amine compound may be used.

Examples of the amino compound include low molecular weight compounds including monoamines such as methylamine, ethylamine, propylamine, butylamine, hexylamine, octylamine, monoethanolamine, diethanolamine, dimethylamine, diethylamine, diisopropylamine, dibutylamine, 2-amino-2-methylpropanol, and the like; diamines such as ethylenediamine, 1,2-diaminopropane, 1,3-diaminopropane, 1,4-diaminobutane, 1,6-diaminohexane, 1,8-diaminooctane, 1,2-diaminocyclohexane, dimer acid amides, N,N'-bis(2-aminoethyl)ethylenediamine, N,N'-diaminopropane, N,N'-bis(3-aminopropyl)ethylenediamine, N,N'-dimethyldiaminopropane, N,N'-dimethyldiaminooctane, and the like; triamines such as dicyandiamide, 1,2,3-triaminopropane, 1,2,3-triamino-2-methylpropane, 1,3-diamino-2-aminomethylpropane, 1,2-diamino-2-aminomethylbutane, 1,3-diamino-2-methyl-2-aminomethylpropane, tris(2-aminoethyl)ethane, tris(6-aminohexyl) isocyanurate, 1,3-diamino-2-methylaminopropane, 2-amino-1,3-bis(isopropylamino)-2-methylpropane, 2-amino-1-isopropylamino-2-isopropylaminomethylbutane, and the like; tetramines such as tetrakis(aminomethyl)methane, tetrakis(methylaminomethyl)methane, tetrakis(2-aminoethylaminomethyl)methane, 1,1,1-tris(2-aminoethylaminomethyl)ethane, and the like; polyalkylene polyamines such as diethylenetriamine, triethylenetetramine, tetraethylenepentamine, hexaethyleneoctamine, nonaethylenedecamine, 1,3-bis(2-aminoethylamino)propane, triethylene-bis(trimethylene)hexamine, bis(3-aminopropyl)amine, 1,3-bis(3-aminopropylamino)propane, spermidine, homospermidine, N-(4-aminobutyl)cadaverine, bis(5-aminopentyl)amine, spermine, 1,6-bis(2-aminoethylamino)hexane, 1,10-bis(2-aminoethylamino)decane, and the like; alicyclic amines such as pyrrolidine, piperidine, piperazine, morpholine, thiomorpholine, and the like; basic amino acids such as lysine, ornithine, arginine, and the like; aromatic amines such as aniline, diphenylamine, and the like; aralkylamines such as benzylamine, and the like; basic heterocyclic compounds containing a nitrogen atom(s) such as pyrrole, imidazole, thazole, and the like; and the like. Among these, ethylenediamine, 1,3-diaminopropane, and N,N'-dimethyldiaminopropane are preferably used; and more preferably used are 1,6-diaminohexane, and N,N'-dimethyldiaminooctane.

The amino compound is usually used singly, but two or more compounds may be used in combination.

In the following, a method for preparing the aqueous dispersion, which is a component of the composition according to the present invention, is described. The aqueous dispersion, a component of the composition according to the present invention, is provided by means of dispersing a compound containing at least one 5-membered ring dithiocarbonate group and an ether bond, into water.

The compound containing at least one 5-membered ring dithiocarbonate group and an ether bond within its molecule as starting material, is provided by means of reacting a compound containing a hydroxyl group with epichlorohydrin to synthesize a glycidyl ether, and then reacting the glycidyl ether, if necessary in the presence of a reaction solvent, with carbon disulfide in the presence of an alkali halide such as lithium bromide, according to a method in accordance with the known method (Japanese Published Unexamined Patent Application No. 247027/93).

For example, the compound expressed by the general formula (II) may be provided by means of reacting a corresponding epoxy resin, which is commercially available, with carbon disulfide in the presence of an alkali halide such as lithium bromide, in the aforementioned manner. Additionally, although the commercially available epoxy resin, as starting material, may be used, if not commercially available, the epoxy resin may be provided by means of reacting a compound containing a hydroxyl group with epichlorohydrin in the presence of a base, according to a known method.

Furthermore, it is possible to provide the aqueous dispersion by means of adding water to the compound containing at least one 5-membered ring dithiocarbonate group and an ether bond, obtained in the aforementioned manner; and if necessary, dissolving the compound completely in a solvent, and further stirring the mixture. In this step, a surfactant, a coalescing agent, a plasticizer, and the like, may be added, if necessary. In addition, after completing stirring, a reaction solvent may be removed under reduced pressure, if necessary. Furthermore, when using water as a reaction solvent, the reaction solution may be used without modification as an aqueous dispersion.

The solvent used in this step is not particularly limited; however, examples of the solvent include benzene, toluene, xylene, hexane, cyclohexane, ethyl acetate, butyl acetate, 3-methyl-3-methoxybutyl acetate, ethylene glycol monobutyl ether acetate, acetone, methyl ethyl ketone, methyl isobutyl ketone, diisobutyl ketone, cyclohexanone, methanol, ethanol, propanol, isopropanol, butanol, isobutanol, N-methylpyrrolidone, tetrahydrofuran, acetonitrile, ethylene glycol monobutyl ether, ethylene glycol monobutyl ether acetate, propylene glycol monomethyl ether, dipropylene glycol monomethyl ether, propylene glycol monoethyl ether, propylene glycol monomethyl ether acetate, methoxybutanol, methoxybutyl acetate, 3-methyl-3-methoxy-1-butanol, water, dimethylsulfoxide, dimethylformamide, dimethylacetamide, and the like.

Examples of the surfactant include anionic emulsifiers such as salts of alkylbenzene sulfonates, sulfates of higher alcohols, polyoxyethylene alkyl sulfates, and the like; nonionic emulsifiers such as polyoxyethylene alkylphenol ethers, sorbitan derivatives, and the like; reactive emulsifiers such as "Eleminol JS-2" (which is manufactured by Sanyo Chemical Industries, Ltd.), "Aqualone HS-10", "Aqualone HS-20" (both of which are manufactured by Dai-ichi Kogyo Seiyaku Co., Ltd.), and the like; high polymer emulsifiers which contain a hydrophilic group such as s salt of a carboxyl group, a salt of a sulfonic acid group, or the like within various polymers (e.g., vinyl polymer, polyester, and the like); and the like. Additionally, a protective colloid such as polyvinyl alcohol, cellulose, or the like, may be used together, for stabilizing the emulsification process.

The composition according to the present invention is characterized by comprising an aqueous dispersion containing at least one 5-membered ring dithiocarbonate group and an ether bond within its molecule, and an amino compound; the composition can be provided by means of dispersing and mixing the aforementioned uniformly.

Furthermore, the composition according to the present invention is conceived to include from the composition, at the time that an amino compound is added to the aqueous dispersion, and curing begins, to the composition, in which the aforementioned has completed curing.

In addition, depending on the objects and the use, various plasticizers, and/or coalescing agents may be added to the composition according to the present invention.

In the composition according to the present invention, the equivalent ratio of the components, the amino compound to the compound containing at least one 5-membered ring dithiocarbonate group, may be 0.3 to 2.0 as the ratio of the amino group to the 5-membered ring dithiocarbonate group, preferably 0.8 to 1.2, as it provides excellent film properties.

The composition according to the present invention may be used by itself as clear paints or clear inks. Furthermore, by adding a pigment to the composition, this composition may be also used as color paints or color inks. As a method for dispersing a pigment, known dispersing agents such as a paints shaker, a ball mill, and the like, may be used; alternatively, a commercially available processed pigment, which has been already dispersed, may be used.

In addition, the composition according to the present invention may be blended with various kinds of UV absorbers, antioxidants, hindered amine light stabilizers, antifoamers, thickeners, flash rust inhibitors, or the like, if necessary. Furthermore, if necessary, the composition according to the present invention may be blended with various, conventionally employed resins such as water soluble alkyd resins, water soluble acrylic resins, water soluble polyurethane resins, water soluble acrylic modified polyurethane resins, water soluble acrylic modified epoxy resins, or styrene acrylic emulsion polymers.

When the composition according to the present invention is used in paints, this composition can be used as a one-liquid type paint by stably dispersing the compound containing at least one 5-membered ring dithiocarbonate group in water and blending the amino compound, or as a two-liquid type paint comprising a combination of the aforementioned compound and the amino compound, and can be adjusted depending on the purpose. In addition, also when the composition according to the present invention is used in an adhesive, this composition can be used as a one-liquid type adhesive of the aforementioned compound with the amino compound, or as a two-liquid type paint comprising a combination of the aforementioned compound and the amino compound.

A painting method for the paint according to the present invention include conventional painting methods such as brush coating, spraying, and the like. As the condition for curing, a wide variety of conditions from drying at ordinary temperature to drying by heating can be chosen. Types of materials to be painted include metal, wood, plastics, inorganic raw material, concrete, asphalt, and the like; and as an under coating material, top coating material, or one-coat finisher, the paint according to the present invention is useful in protecting raw materials and improving coating appearance, and the like.

The composition according to the present invention possesses superior properties in hardness, strength, adhesive property, water resistance, chemical resistance, stain resistance, blocking resistance, and the like.

In the following, the present invention is explained using the examples and reference examples.

### Best Mode for Carrying Out the Invention

### Reference Example 1

In a flask equipped with a stirrer, thermometer, dropping apparatus, condenser tube, and nitrogen gas introduction tube, a solution was prepared by dissolving 500 ml of tetrahydrofuran, 50 g of a bisphenol A epichlorohydrin epoxy resin ("YD-901"; manufactured by Tohto Kasei Co., with a styrene standard converted number-average molecular weight of 974), and 2.5 g of lithium bromide. Carbon disulfide (70 ml) was added dropwise to the solution at 25 °C, the mixture in the flask was heated to 45°C (internal temperature), and the contents were allowed to react for 10 hours. After finishing the reaction, this solution was concentrated under reduced pressure. Subsequently, 300 ml of chloroform, 200 ml of acetone, and 500 ml of water were added to the concentrated residue obtained to perform separation. Saturated brine (500 ml) was then added to the extracted organic layer, and the separation was repeated. After dehydrating the resultant organic layer with 15 g of magnesium sulfate, the solvent was removed to yield 90 g of a crude product.
¹H-NMR and FT-IR analyses of the resultant product yielded the following results:
¹H-NMR (CDCl₃, ppm, 400MHz): 1.63 (s, 18H), 2.52 (d, J=5.1Hz, 2H), 3.73 (dd, J=7.1, 12.0Hz, 4H), 4.13 (dd, J=4.6, 8.0Hz, 4H), 4.25 (dd, J=5.6, 10.3Hz, 2H), 4.30 (dd, J=5.6, 10.3Hz, 2H), 4.35 (q, J=5.4Hz, 2H), 5.42 (m, 2H), 6.81 (dd, J=1.7, 8.8Hz, 6H), 6.83 (dd, J=2.0, 8.8Hz, 6H), 7.13 (dd, J=1.5, 8.0Hz, 12H)
IR(NaCl, cm⁻¹): 508, 1184, 1241, 1606, 3037, 3442

### Reference Example 2

The product obtained (10 g) in Reference Example 1,3.0 g of toluene, 0.6 g of ethylene glycol monobutyl ether, 1.0 g of a nonionic emulsifier "Emulgen 931" (which is manufactured by Kao Corp.), and 0.3 g of a nonionic emulsifier "Emulgen 930" (which is manufactured by Kao Corp.) were put into a flask equipped with a stirrer, thermometer, dropping apparatus, condenser tube, and nitrogen gas introduction tube, and the internal temperature of the flask was raised to 50°C. After uniformly stirring the solution, 18 g of warm water, which had been pre-heated to 50°C, was added dropwise to the aforementioned solution, to yield a resin solution (A-1) with a solid content of 34% by weight, and a viscosity of 780 cps.

### Reference Example 3

A bisphenol A epichlorohydrin epoxy resin, 10 g ("YD-901"; manufactured by Tohto Kasei Co.), 3.0 g of toluene, 0.6 g of ethylene glycol monobutyl ether, 1.0 g of a nonionic emulsifier "Emulgen 931" (which is manufactured by Kao Corp.), and 0.3 g of a nonionic emulsifier "Emulgen 930" (which is manufactured by Kao Corp.) were put into a flask equipped with a stirrer, thermometer, dropping apparatus, condenser tube, and nitrogen gas introduction tube, and the internal temperature of the flask Was raised to 50°C. After uniformly stirring the solution, 18 g of warm water, which had been preheated to 50°C, was added dropwise to the aforementioned solution, to yield a resin solution (B-1) with a solid content of 34% by weight, and a viscosity of 780 cps.

### Example 1

1,6-diaminohexane (4.0 g) was added to 100 g of the resin solution (A-1) obtained in Reference Example 2, to yield a clear varnish solution.

### Comparative Example 1

1,6-diaminohexane (4.0 g) was added to 100 g of the resin solution (B-1) obtained in Reference Example 3, to yield a clear varnish solution.

### Test Example 1

Test pieces were prepared by applying clear varnish solutions obtained by Example 1 and Comparative Example 1 to cold-finished steel plates (manufactured by Japan Test Panel Co. (Osaka), Ltd.), which were treated with iron phosphate, using an applicator coater so that the thickness of the films after drying would be 30 □m, and drying them at room temperature for a week. The following tests were conducted on the test pieces.
Gel fraction: Approximately 0.5 g of the film was peeled, sampled from a test piece, and washed under acetone reflux for 8 hours by a Soxhlet extractor. Subsequently, the resultant product was dried under reduced pressure overnight, and the remaining rate by weight was calculated to determine the gel fraction.
Pencil hardness: Tests were conducted by the manual scratch method according to JIS K5400 (General Test Method for Coatings).
Adhesion: Tests were conducted by the cross-cut adhesion test method according to JIS K5400 (General Test Method for Coatings).
Impact resistance: Tests were conducted by dropping a 500 g weight on a test piece, and measuring the height required to cause peeling of the coating film, using a Du Pont impact tester according to JIS K5400 (General Test Method for Coatings).
Methyl ethyl ketone (MEK) resistance: A cloth was soaked with MEK, and tests were conducted by rubbing a test piece back and forth with the cloth under a load of 500 g. The number of rubbing strokes was counted until the article coated became exposed.

In addition, in order to test the storage stability at the time of blending the amine, 70 ml of the prepared, clear varnish was stored in 100 ml of a glass bottle for 30 days at room temperature. Subsequently, the existence of any abnormality such as gelation was inspected by visual observance.

Table 1 shows the results of the film evaluation tests of the clear varnish solutions obtained in Example 1 and Comparative Example 1.

**Table 1**

| Evaluation items | Example 1 | Comparative Example 1 |
|---|---|---|
| Coating appearance | No abnormality | No abnormality |
| Pencil hardness | H | F |
| Adhesion | 100/100 | 80/100 |
| Impact resistance (cm) | 50 | 10 |
| MEK resistance (No. of times) | 100 | 60 |
| Gel fraction | 92% | 77% |
| Storage stability | No abnormality | Gelated |

Therefore, the aqueous dispersion composition according to the present invention exhibits superior hardness, adhesion, impact resistance, MEK resistance, and storage stability, when compared to the aqueous dispersion composition according to Comparative Example.

### Industrial Applicability

The present invention provides an aqueous dispersion composition which is effective for use in paints, adhesives, inks, sealing compounds for building, sealants for semiconductor, and the like.

## Claims

1. A composition comprising
an aqueous dispersion of a compound containing at least one 5-membered ring dithiocarbonate group, expressed by the following general formula (I): 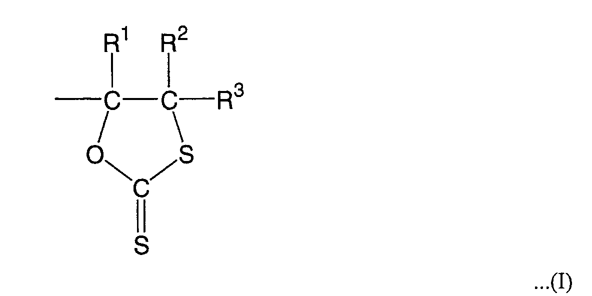 (wherein R¹, R² and R³ are the same or different, and represent a hydrogen atom or a lower alkyl group) and an ether bond; and an amino compound.

2. The composition according to Claim 1, wherein said compound containing at least one 5-membered ring dithiocarbonate group and an ether bond contains phenylene or cyclohexylene within its molecule.

3. The composition according to Claim 2, wherein said compound containing at least one 5-membered ring dithiocarbonate group and an ether bond, and containing phenylene or cyclohexylene within its molecule is a compound expressed by the following general formula (II): 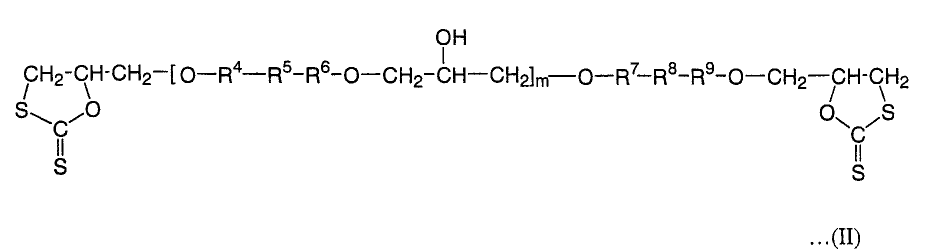 (wherein R⁴, R⁶, R⁷ and R⁹ are the same or different, and represent phenylene or cyclohexylene, in which 1 to 4 hydrogen atoms may be substituted with Br; R⁵ and R⁸ are the same or different, and represent methylene, C(CH₃)₂, or S; and m represents an integer between 1 and 40).

4. The composition according to Claim 3, wherein R⁴, R⁶, R⁷ and R⁹ represent the same group; and R⁵ and R⁹ represent the same group, in the general formula (II).

5. A paint comprising a composition according to Claim 1.

6. A two-liquid type paint comprising a combination of an aqueous dispersion of a compound containing at least one 5-membered ring dithiocarbonate group, expressed by the general formula (I), and an ether bond; and an amino compound.

7. A two-liquid type adhesive comprising a combination of an aqueous dispersion of a compound containing at least one 5-membered ring dithiocarbonate group, expressed by the general formula (I), and an ether bond; and an amino compound.
